(19) Europäisches Patentamt
European Patent Office
Office européen des brevets

(11) Publication number: **0 002 557**
**B1**

(12) **EUROPEAN PATENT SPECIFICATION**

(45) Date of publication of patent specification: **23.09.81**

(21) Application number: **78200358.6**

(22) Date of filing: **08.12.78**

(51) Int. Cl.³: **B 01 J 31/16**, C 07 B 1/00, C 07 B 29/00, C 07 C 1/02, C 07 C 2/08, C 07 C 27/22, C 07 C 45/50

# (54) Resin-ligand transition metal complex composition and processes for using this composition as catalyst.

(30) Priority: **19.12.77 US 861916**

(43) Date of publication of application:
**27.06.79 Bulletin 79/13**

(45) Publication of the grant of the European patent:
**23.09.81 Bulletin 81/38**

(84) Designated Contracting States:
**BE DE FR GB IT NL**

(56) References cited:
**DE - A - 2 633 959**
**FR - A - 2 153 421**
**FR - A - 2 270 238**
**GB - A - 1 238 703**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Kim, Leo**
**12126 Westmere Drive**
**Houston Texas 77077 (US)**
Inventor: **Paxson, Timm Edward**
**15910 Haven Hills Drive**
**Houston Texas 77084 (US)**
Inventor: **Tang, Sunny Conrad**
**20231 Laurel Lock Drive**
**Katy Texas 77450 (US)**

(74) Representative: **Keuzenkamp, Abraham et al,**
**P.O. Box 302**
**NL-2501 CH Den Haag (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Resin-ligand transition metal complex composition and processes for using this composition as catalyst

This invention relates to a complex composition comprising an ion-exchange resin with a ligand ionically bound thereto and with the ligand coordinately bound to a transition element. Such complex composition is useful as a heterogenous catalyst in organic reactions.

The use of heterogenous catalysts over homogeneous catalysts has several advantages, such as allowing the use of fixed beds, ease of separation of catalyst from the product and catalyst recovery and regeneration.

Traditionally, to produce heterogeneous catalysts from metals of the transition element series, these metals have been deposited on inert supports, such as alumina or silica. More recently metal catalysts have been covalently attached to inert resin backbones by use of diphenyl phosphine or other ligands which are attached directly to the polymer and coordinately bound to the metal. Typical examples of this type are found in U.S. Patent 3,998,864 and in Pittman et al., *Chemtech.*, p.560—566, 1973.

There has now been found a novel type of heterogeneous catalyst which is a complex composition comprising:

(a) an ion exchange resin;
(b) a transition metal and
(c) an organic linking compound which has at least one moiety which is ionically bound to said ion-exchange resin and further has at least one moiety which is coordinately bound to said metal.

In the composition of the invention, the metal is coordinately bound to a ligand and the ligand is ionically bound to an ion-exchange resin. Some of the advantages of the materials of the invention are that the materials are relatively simple to prepare using commercially available compounds, the preparations involve no exotic conditions, and often may be carried out in an aqueous solvent system and the resins may be easily stripped of metal and ligands for isolation of the metal species and regeneration of the catalyst. The resin-based catalysts of this invention have unique selectivity-reactivity properties when compared to their homogeneous analogues.

The ion-exchange resins utilized in the composition of this invention are well known in the art and are readily available commercially. These are in the gel form or are macroporous and are either strongly acidic, weakly acidic, strongly basic, intermediately basic, weakly basic or are of a mixed acid-base type. The strongly acidic resins typically are resins of cross-linked styrene, styrene-divinylbenzene, phenol-formaldehyde, benzene-formaldehyde, having functional sulphonic or phosphonic acid groups attached thereto. Also suitable are the fluorinated alkyl sulphonic acid resins containing the —$CFSO_3H$ group as, for example, the NAFION® type resins supplied by E.I. Du Pont de Nemours. The weakly acidic resins are those with carboxylic acid groups and are typically acrylic acid derivatives, such as, for example, those resins prepared by the copolymerization of methacrylic acid and divinyl benzene. Another weakly acidic resin is the chelating type which is a styrene-divinyl benzene copolymer containing iminocarboxylic acid groups such as iminodiacetic acid functional groups which can serve as an anion exchanger at very low pH. The basic resins typically are resins of cross-linked styrene, styrene-divinyl benzene, phenol-formaldehyde, benzene-formaldehyde, epoxypolyamine, phenolic-polyamine having functional amine, either primary, secondary, tertiary or quaternary, or pyridinium groups attached thereto. Typical examples of suitable commercially supplied resins are given in Table 1 of Bio-Rad Laboratories Catalogue, Chromatography, Electrophases, Immunochemistry and Membrane Filtration, Price List C, March, 1977.

The preferred resin choice for the composition of this invention will depend on the particular ionically bondable moiety utilized on the linking compound as well as on the particular use envisioned for the composition. For example, if the composition were used in a liquid-phase catalysis, the composition and pH of the liquid would determine the preferred resin to be utilized.

The linking compound is hydrocarbyl, i.e., alkyl, aryl or mixtures of aryl and alkyl which alkyl can be either cyclic or acyclic or mixtures thereof, containing from 1 to 100 carbon atoms, preferably from 3 to 80 carbon atoms and has at least two moieties containing an atom other than carbon or hydrogen.

At least one moiety of the linking compound is in the ionic or ionizable form and is compatible with the exchange group on the ion-exchange resin, i.e., when the exchange group is acidic, the resin-compatible ionic moiety on the linking compound is basic-derived and vice versa. For basic-type resins the resin-compatible ion moiety is derived from carboxylic acid ($RCO_2^-$), phosphonic acid ($RPA(OH)O^-$), phosphinic acid ($R_2POO^-$, sulphenic acid ($RSO^-$), sulphinic acid ($RSOO^-$), sulphonic acid ($RSO_2O^-$), boronic acid ($RB(OH)O^-$), boronous acid ($RBO^-$). For acidic or mixed acid-base-type resins the resin-compatible ion moiety is monohydrocarbyl ammonium ($RN^+H_3$), dihydrocarbyl ammonium ($R_2N^+H_2$), trihydrocarbyl ammonium ($R_3N^+H$), quaternary ammonium ($R_4N^+$), pyridinium ($RC_5H_4N^+R_1$), phosphonium ($R_4P^+$), arsonium ($R_4As^+$) and sulphonium ($R_3S^+$).

The linking compound may have more than one of the ionic moieties. It may be polyfunctional, for

example, in carboxylate ion, in phosphonate ion, in sulphonate ion, in quaternary ammonium ion or in pyridinium. The polyfunctional group may be the same or different.

At least one other moiety of the linking compound has an atom capable of complexing with transition metals, and consists of trivalent nitrogen, trivalent phosphorus, trivalent arsenic trivalent bismuth, and trivalent antimony.

The three valences of the complexing atoms may be satisfied by any organic radical: saturated or unsaturated aliphatic and/or saturated or unsaturated heterocyclic and/or aromatic radicals. These radicals may contain any functional group, such as carbonyl, nitro, and hydroxy groups as well as saturated and unsaturated alkyl groups and the radical may be bound to the complexing atom directly through a carbon-complexing atom linkage or through an electro-negative atom, such as oxygen or sulphur.

It is also suitable for a simple organic radical to satisfy more than one of the valence of the complexing atom, thereby forming a heterocyclic compound with the trivalent complexing atom. For example, an alkylene radical may satisfy two of the valences thereby forming a cyclic compound. Another example would be the alkylene dioxy radical to form a cyclic compound where oxygen atoms link an alkylene radical to the complexing atom. In these two examples the third valence may be satisfied by any other organic radical.

The linking compound may have more than one of the metal complexing moieties, It may be, for example, polydentate in phosphorus atom, e.g., it may be bi- or tridentate, having two or three phosphorus atoms. It may have mixed complexing atoms, e.g., a phosphorus and arsenic atom or two phosphorus atoms and one nitrogen atom, etc.

The trivalent nitrogen atom will be present as an amine, i.e., as a primary, secondary, tertiary, quaternary amine or as pyridine or cyanide. The trivalent phosphorus will be present as phosphine ($R_3P$), phosphinite ($ROPR_2$), phosphonite ($(RO)_2PR$ and phosphite ($RO_3P$). Correspondingly, trivalent arsenic will be available as arsine, arsinite, arsonite and arsenite; trivalent bismuth as bismuthine, bismuthinite, bismuthonite and bismuthite; and trivalent antimony as stibine, stibinite, stibonite and stibite. The preferred complexing atoms are phosphorus and nitrogen. The tertiary amines, phosphines, arsines and stibines and bismuthines have a marked tendency to form non-ionic complexes with metals.

When the linking compound is polydentate in an ionizable hetero-atom, it is understood that there will be a statistical distribution of the ionized atoms upon quaternization or protonation. For example, if one mole of a linking compound which contains 3 amine groups, is protonated with 2 moles of HCl, then some of the molecules of the linking compound will have 3 quaternized amine groups, some will have 2 and some will have 1, but on the average there will be 2 quaternized amino groups per molecule. It is further understood from general principles of organic chemistry that unit charges resulting from quaternization and protonation can be distributed as partial charges over several heteroatoms in a linking compound molecule.

Thus, the linking compound as reacted in the composition of this invention will have at least one protonized or quaternized hetero-atom and at least one hetero-atom complexed with a transition metal. Suitable linking compounds utilized in making the composition of the invention include but are not limited to the following examples:

tris(dimethylamino)phosphine
tris(diethylamino)phosphine
tris(di-isopropylamino)phosphine
tris(methylethylamino)phosphine
tris(p-dimethylaminophenyl)phosphine
tris(p-diethylaminophenyl)phosphine
tris(p-methylethylaminophenyl)phosphine
tris(o-dimethylaminophenyl)phosphine
tris(m-dimethylaminophenyl)phosphine
tris(dimethylaminoethyl)phosphine
ethyl-bis(diphenylphosphinoethyl)amine.

Substitution of phosphinites, phosphonites, phosphites for the phosphine in the above compounds as well as arsines, arcinites, arsonites, arsenites, bismuthines, bismuthinites, bismuthonites, bismuthites, stibines, stibinites, stibonites, stibites and amines produces linking compounds useful in preparing the compositions of this invention. Other suitable compounds are:

2-(P,P-diphenylphosphino)benzoic acid
tris(beta-aminoethyl)amine
nicotinic acid
isonicotinic acid
2-chloronicotinic acid
1,1-dimethyl-4-phenyl piperazinium salt

2,2'-alkylarsino-1,1'-diphenylamine
2-(P,P-dicyclohexylphosphino)benzoic acid
beta-(dicyclohexylphosphino)propionic acid
1,4-(P,P-diphenylphosphino)benzene
2-diphenylphosphino-3-carboxy-2-butene
2-(P,P-diphenylphosphino)benzene sulphonic acid
2-amino-s-triazine
1-diphenylphosphino-2-diphenylphosphinoethane
bis-(diphenylphosphinoethyl)ethylamine
3-dialkylphosphino)benzene phosphonic acid.

The preferred metals complexed with the linking compound are selected from the transition elements of the Periodic Table: Groups IVB, VB, VIB, VIIB, V, VIII, IB and IIB, technetium excluded. The metals are:

TABLE I

| IVB | VB | VIB | VIIB | VIII | IB | IIB |
|---|---|---|---|---|---|---|
| Ti | V | Cr | Mn | Fe Co Ni | Cu | Zn |
| Zr | Nb | Mo | — | Ru Rh Pd | Ag | Cd |
| Hf | Ta | W | Re | Os Ir Pt | Au | Hg |

More preferred metals are from Groups VIB, VIIB, VIII and IB.

The complexed metals can be in various oxidation states. See "Complexes of the Transition Metals with Phosphines, Arsines and Stibines" by G. Booth, Adv. Inorg. Nucl. Chem., *6*, 1—69 (1964) for a comprehensive description of complexes. For example, the Booth reference cites the following oxidation states for metals complexed with phosphines.

TABLE II

| Metal | Oxidation state for stable phosphine complexes |
|---|---|
| Ti | 4 |
| Zr | 4 |
| Hf | 4 |
| V | 0, 3, 4 |
| Cr | 0, 2, 3 |
| Mo | 0, 1, 2, 3, 4 |
| W | 0, 1, 2, 3, 4 |
| Mn | 0, 1 |
| Re | 0, 1, 2, 3, 4, 5 |
| Fe | 0, 1, 2, 3 |
| Ru | 0, 2, 3, 4 |
| Os | 2, 3, 4 |
| Co | 1, 2, 3 |
| Rh | 0, 1, 3 |
| Ir | 1, 3 |
| Ni | 0, 1, 2, 3 |
| Pd | 0, 2 |
| Pt | 0, 2 |
| Cu | 1, 3 |
| Ag | 1 |
| Au | 1, 3 |

Articles dealing with the complexing of amines with metals are "Inorganic Complexes", Jorgensen, C.K., Academic Press 1963, Chapter 4 and "Chemistry Coordination Compounds," Bailer (Ed.) Am. Chem. Soc. Monograph Series 131, 1956. The above references cite the following oxidation states for metals complexed with amines.

TABLE III

| Metal | Oxidation state for stable amine complexes |
|---|---|
| Cr | 0, 1, 2, 3 |
| Mo | 0, 3 |
| W | 2 and 3 (polynuclear)<br>4 (mononuclear) |
| Mn | 2 |
| Re | 3, 5 |
| Fe | 2, 3 |
| Ru | 2, 3 |
| Os | 2, 3, 4 |
| Co | 2, 3 |
| Rh | 3 |
| Ir | 3, 4 |
| Ni | 0, 2 |
| Pd | 2 |
| Pt | 0, 2, 4 |
| Cu | 2 |
| Ag | 1, 2 |
| Au | 3, |

The composition according to the invention may have more than one transition metal present. The composition may also have the metal(s) co-complexed with other ligands in addition to the linking compound. For example, from the above-noted Booth reference the metal complexed moiety of the composition could have the following form and still be within the scope of the invention, i.e.:

$$M_Y\ M'_Z\ O_A\ H_B\ X_C\ (CN^-)_D\ (CO)_E(NO)_F(Cp)_G\ (Py)_H(Acac)_I(AsR_3)_J(NR_3)_K\ (PR_3)_L(SnX_3^-)_M\ (GeX_3^-)_M(Carb)_NP_Q$$

$M_Y$ = metal in oxidation state shown in Table II or Table III
Y = zero to n mononuclear to polynuclear cluster
$M'_Z$ = metal in oxidation state shown before
Z = zero to n mononuclear of mixed metal polynuclear cluster where n is an integer greater than zero when Y > zero and Z > zero
O = oxygen where
A = zero to n
H = hydrogen where
B = zero to n
X = halide, F, Cl, Br, I; where
C = zero to 5
$(CN^-)$ = cyanide where
D = zero to 5 when y + z = 1 or D = 1 to n when y + z > 1
(CO) = carbonyl where
E = zero to 5 when y + z = 1 or E = 1 to n when y + z > 1
(NO) = nitrosyl where
F = zero to 5 when y + z = 1 or F = 1 to n when y + z > 1
Cp = cyclopentadienyl where
G = zero to 3 when Y + z = 1 or G = 1 to n when y + z > 1

Py = pyridine where
H = zero to 5 when $y+z=1$ or $H=1$ to n when $y+z>1$
Acac = acetylacetonate where
I = zero to 3 when $y + z = 1$ or $I = 1$ to n when $y + z > 1$
$(AsR_3)$ = arsines, where R = H, alkyl or aryl and
J = zero to 5 when $y+z=1$ or $J=1$ to n when $y+z>1$
the arsine also may be of the chelating type or contain mixed donating atoms, e.g.:

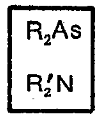
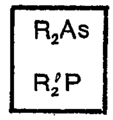

$(NR_3)$ = amines, where R = H, alkyl, or aryl and K = zero to 5 when $y + z = 1$ or $K = 1$ to n when $y + z > 1$ as with arsines, a chelating or mixed donor chelating ligand may be employed.

$(PR_3)$ = phosphines where R = H, alkyl, or aryl, and L = zero to 5 when $g+z=1$ or $L=1$ to n when $y+z>1$ as with arsines, and amines, a chelating ligand may be employed.

$(SnX_3^-)$ or $(GeX_3^-)$ = trihalostannyl or trihalogermyl where X = F, Cl, Br, I and M = zero to 5 when $y+z=1$ or $M=1$ to n when $y+z>1$

(Carb) = carboxylate where N = zero to 5 when $y+z=1$ or $N=1$ to n where $y+z>1$

P = the bridging moiety/ligand between the metal and the resin support and Q = 1 to n.

The compositions of the invention find use as catalysts in many chemical processes. Illustrative examples are the use of compositions containing rhodium or ruthenium complexes in hydroformylation, carbonylation, hydrogenation, isomerization and Fischer-Tropsch reactions. Compositions containing cobalt complexes are useful in hydroformylation, carbonylation, hydrogenation and isomerization reactions. Compositions containing molybdenum complexes are used in disproportionation (metathesis) and isomerization reactions. Compositions containing palladium and platinum complexes are useful in hydroformylation, carbonylation, isomerization, hydrogenation and oligomerization/dimerization reactions. Compositions containing nickel complexes are useful in ologomerization/dimerization reactions. Compositions containing tungsten or rhenium complexes are useful in metathesis reactions. Olefins are hydroformylated with hydrogen and carbon monoxide and they are carbonylated with carbon monoxide and a hydroxylic compound.

The catalyst preparation procedures described below were carried out in nitrogen-filled dry boxes. The solvent benzene was purified by distillation over $CaH_2$, all other solvents were of reagent-grade and used as supplied. The metal complexes

$[Rh(CO)_2Cl]_2$, $Rh(PPh_3)_3CL$, $Rh(NO_3)_3$ in $H_2O$, $Co_2(CO)_8$,

$Ru(PPh_3)_3Cl_2$, $Mo(NO_2)Cl_2$, $PtCl_2(PPh_3)_2$, $[PtCl_2(PBu_3)]_2$,

$Pd(C_6H_5CN)_2Cl_2$, $PdCl_2(PPh_3)_2$, $[Pd(allyl)Cl]_2$ and $Pd(OAc)_2$,

and compound $CH_3Br$, $SnCl_2.2H_2O$, iso-nicotinic acid, and phosphines

$[(CH_3)N]_3P$, $[(CH_3)_2NC_6H_4]_3P$, $[(CH_3)_2NCH_2CH_2O]_3P$

were used as supplied. The quaternized aminophosphines were prepared by the reaction of 1 equivalent of $CH_3Br$ with an aminophosphine in toluene solution at room temperature. The quaternized aminophosphine precipitated readily from the toluene solution. The complex $Ru(CO)_3(PPh_3)_2$ (J. Chem. Soc. Dalton, 399 (1976)), $PtH(SnCl_3)(PPh_3)_2$ and $PtH(SnCl_3)(CO)(PPh_3)_2$ (J. Am. Chem. Soc. *97*, 3553 (1975)) were prepared as described in the references. The resins are indicated by (resin backbone)-(exchange group), e.g., a sulphonated styrene divinylbenzene resin would be (styrene divinyl benzene)-$(SO_3^-)$, etc. Ph, $C_6H_5$ and $\varphi$ are used as abbreviations for phenyl; —$\varphi$— and $C_6H_4$ indicate p-substituted benzene moieties.

*Preparation of resin-linking compound moiety*

Example 1

Preparation of sulphonated styrene-divinylbenzene resin/$[(CH_3)_2N]_3$ P compound.

The aminophosphine $[(CH_3)_2N]_3P$ (0.98 g, 60 mmol.) was dissolved in 175 ml of acetone in a 250

ml round-bottomed flask. 5.0 grams of Rohm and Haas XN1010H$^+$ resin (acid form; macroreticular sulphonated styrene-divinylbenzene, 3.3 meq./g) which had previously been thoroughly washed with de-ionized water and dried were added to the flask. The mixture was then stirred magnetically from the side of the flask for 48 hours to prevent resin attrition. The resin was filtered by suction, washed with 3 x 50 ml of acetone, and dried in vacuum oven (50°C) overnight. Analysis showed the product as having the approximate formula

$$(\text{styrene-divinylbenzene})\text{-}(SO_3^-)_{1.5}\ [([(CH_3)_2N]_3P)(H^+)_{1.5}].$$

Example 2

Preparation of sulphonated styrene-divinylbenzene resin/$[(CH_3)_2NC_6H_4]_3P$ compound

The aminophosphine $[(CH_3)_2NC_6H_4]_3P$ (14.0 g, 35.8 mmol.) was dissolved in 1000 ml warm benzene, cooled to room temperature, and filtered into a 2-litre round-bottomed flask quickly. 10.0 g of XN1010H$^+$ ion-exchange resin were added, and the mixture stirred magnetically on side of flask for 72 hours. The resin was then filtered, washed with benzene and vacuum-dried in oven (40°C). Analysis showed the product as having the approximate formula

$$(\text{styrene-divinylbenzene})\text{-}(SO_3^-)_{1.5}[([(CH_3)_2NC_6H_4]_3P)(H^+)_{1.5}]$$

Example 3

Preparation of sulphonated styrene-divinylbenzene resin/$[(CH_3)_2NCH_2CH_2O]_3P$ compound

This material was prepared in a similar manner as in Example 1 except that 3.54 g (12.0 mmol.) of the aminophosphone $[(CH_3)_2NCH_2CH_2O]_3P$. 10.0 g of XN1010H$^+$ resin, and 300 ml of acetone were used. Analysis showed the product as having the approximate formula

$$(\text{styrene-divinylbenzene})\text{-}(SO_3^-)_{1.5}[([(CH_3)_2NCH_2CH_2O]_3P)(H^+)_{1.5}].$$

Example 4

(Preparation of sulphonated styrene-divinylbenzene resin/$[\varphi_2PCH_2CH_2)_2NCH_2CH_3]$ compound

This material was prepared in a similar manner to Example 1 except that 2.82 g (6 mmol.) of the aminophosphine $(\varphi_2PCH_2CH_2)_2NCH_2CH_3$ and 200 ml of acetone were used. Analysis showed the product as having the approximate formula

$$(\text{styrene-divinylbenzene})\text{-}(SO_3^-)_{1.5}\ [([(C_6H_5)_2(PCH_2CH_2]_2NCH_2CH_3)(H^+)_{1.5}].$$

Example 5

Preparation of sulphonated styrene-divinylbenzene resin/methyl-quaternized $[CH_3)_2N]_3P$ compound

The quaternized aminophosphine $([CH_3)_2N]_3P)(CH_3^+)Br^-$ (7.0 g wet) was dissolved in 350 ml of de-ionized water in a 500 ml round-bottomed flask. 10.0 g of XN1010Na ion-exchange resin (prepared by exhaustive ion-exchange of XN1010H$^+$ with 10 litres of 1N NaCl or when the pH of the effluent wash was neutral) were added. The mixture was side-stirred for 48 hours, filtered with suction, and the resin washed with 5 x 100 ml deionized $H_2O$, then vacuum-dried in oven overnight (45°C). Analysis showed the product as having the approximate formula

$$(\text{styrene-divinylbenzene})\text{-}(SO_3^-)\ [([(CH_3)_2N]_3P)(CH_3^+)].$$

Example 6

Preparation of sulphonated styrene-divinyl benzene resin/methyl-quaternized $([(CH_3)_2NC_6H_4]_3P)$ compound

This material was prepared in a similar manner as in Example 5 except that 10.4 g (21.1 mmol.) of the quaternized aminophosphine, 12.0 g of XN1010Na, and 1900 ml of an acetone/$H_2O$ (12:7 v/v) solution was used. Analysis showed the product as having the approximate formula

$$(\text{styrene-divinylbenzene})\text{-}(SO_3^-)\ [([(CH_3)_2NC_6H_4]_3P)(CH_3^+)].$$

Example 7

Preparation of sulphonated styrene-divinylbenzene resin/methyl-quaternized $[(CH_3)_2NCH_2CH_2O]_3P$ compound

This material was prepared in a similar manner as in Example 5 except that 5.6 g (14.4 mmol of the quaternized aminophosphine, 8.0 g of XN1010Na, and 400 ml of $H_2O$ were used. Analysis showed the product as having the approximate formula

$$(\text{styrene-divinylbenzene})\text{-}(SO_3^-)\ [([(CH_3)_2NCH_2CH_2O]_3P)(CH_3^+)].$$

Example 8

Preparation of sulphonated styrene-divinylbenzene resin/methyl-quaternized $([(C_6H_5)_2PCH_2CH_2)$ compound

This material was prepared in a similar manner as in Example 5 except that 2.0 g (3.5 mmol.) of the quaternized aminophosphine, 4.0 g of XN1010Na, and 200 ml of de-ionized water were used. Analysis showed the product as having the approximate formula

$$(\text{styrene-divinylbenzene})\text{-}(SO_3^-)\ [([(C_6H_5)_2PCH_2CH_2]_2NCH_2CH_3)(CH_3^+)].$$

Example 9.

Preparation of sulphonated styrene resin/$[(CH_3)_2NC_6H_4]_3P$ compound

This material was prepared in a similar manner as in Example 2 Except that Dowex (registered Trade Mark) MSC-1$H^+$ resin sulphonated polystyrene, macroreticular, 1.6 meq./g) was used. Analysis showed the product as having the approximate formula

$$(styrene)\text{-}(SO_3^-)_{1.5}[([(CH_3)_2NC_6H_4]_3P)(H^+)_{1.5}].$$

Example 10

Preparation of sulphonated styrene resin/methylquaternized ($[(CH_3)_2NC_6H_4]_3P$) compound

50 grams of the hydrogen form of BioRad AG 50w-X1 (sulphonated polystyrene, 5.0 meq./g) were placed in a coarse fritted glass funnel which were then Na-exchanged with 1 litre of 1N NaCl by adding the salt solution in aliquots so it slowly ran through the resin. The resin was then washed in a similar manner with 2 litres of de-ionized water. This was followed by an acetone rinse and the resin was dried in a vacuum oven at about 40°C for 2 days. A 5.0 g portion of the dried Na-form of the resin was then added to 2 litres of an acetone-water solution (1:1 v/v) which contained the quaternized aminophosphine (2.0 g, 4.1 mmol.) and stirred overnight under $N_2$. The material was then filtered and washed with an acetone solution, a water solution and then air-dried. Analysis showed the product as having the approximate formula

$$(styrene)\text{-}(SO_3^-)[([(CH_3)_2NC_6H_4]_3P)(CH_3^+)].$$

Example 11

Preparation of phosphonated styrene-divinylbenzene resin/methyl-quaternized ($[(CH_3)_2NC_6H_4]_3P$ compound

This material was prepared in a similar manner as in Example 10 except that 5.0 g of Bio-Rex (Registered Trade Mark) 63 (micro-reticular (gel), phosphonated, 6.6 meq./g) were used. Analysis showed the product as having the approximate formula

$$(styrene\text{-}divinylbenzene)\text{-}(PO_3^-)\ [([(CH_3)_2NC_6H_4]_3P)(CH_3^+)].$$

Example 12

Preparation of carboxylated acrylic resin/methyl quaternized ($[(CH_3)_2NC_6H_4]_3P$)

This material was prepared in a manner similar to Example 10 except that 5.0 g of Bio-Rex (Registered Trade Mark) 70 (acrylic polymer, carboxylic acid-exchange group, 10.2 meq./g) were used. Analysis showed the product as having the approximate formula

$$acrylic\ (CO_2^-)[([(CH_3)_2NC_6H_4]_3P)(CH_3^+)].$$

Example 13

Preparation of vinyl pyridinium resin/$[o^-\text{-}O_2CC_6H_5P(C_6H_5)_2]$ compound

A 10 g portion of Bio-Rex (Registered Trade Mark) 9 (100—200 mesh, polymerized vinylpyridine, pyridinium type, 3.7 meq./g) was placed in a coarse grade fritted glass funnel and treated with 1 litre of 0.1 N HCl in the manner described in Example 10. The resin was then rinsed with 2 litres of de-ionized water, and finally with acetone. The resin was then dried for 2 days at about 40°C in a vacuum oven.

A 5.0 g portion of resin was then added to about 500 ml of $H_2O$ in a round bottom flask. A 1.29 g (3.8 mmol.) of diphenyl(2-carboxyphenyl)phosphine was placed in a small flask of $H_2O$/acetone (5.0 g $H_2O$, 5.0 ml acetone) with 0.15 g of dissolved NaOH. After the phosphine was dissolved the solution of phosphine was added to the solution which contained the resin. This was stirred overnight under an $N_2$-atmosphere. The material was filtered and washed with a 50—50 v/v mix of acetone/water and finally with acetone. Analysis showed the product having the approximate formula

$$(ethylene)\text{-}(pyridinium^+)\text{-}[o\text{-}O_2CC_6H_5P(C_6H_5)_2^-].$$

Example 14

Preparation of quaternary ammonium styrene-divinylbenzene resin/($HO_2CC_5H_4N$) compound

To a solution of NaOH, to a solution of sodium hydroxide (1.5 g) in 150 ml of de-ionized $H_2O$ were added 4.6 g (38.4 mmol.) of iso-nicotinic acid. To the resultant solution were added 6.0 g of Bio-Rad AG-1 (styrene-divinylbenzene micro-reticular anion-exchange resin, Cl-form, 20—50 mesh, 3.2 meq./g), side-stirred for 2 hours, and filtered by suction. The resin material was then washed with 3 × 50 ml of acetone, and dried in vacuum-oven (45°C). Analysis showed a product having the approximate formula

$$(styrene\text{-}divinylbenzene)\text{-}[CH_2N^+(CH_3)_3](^-O_2CC_5H_4N).$$

Example 15

Preparation of quaternary ammonium styrene resin/($HO_2CC_5H_4N$)

This material was prepared in a similar manner as in Example 14 except that 5.9 g (48.0 mmol.) of iso-nicotinic acid and 6.0 g of the Dowex (Registered Trade Mark) MSA-1 (macroreticular polystyrene base quaternary exchange group, 4.0 meq./g) resin were used. Analysis showed a product having the approximate formula

$(styrene)-[CH_2N^+(CH_3)_3](^-O_2CC_5H_4N)$.

*Preparation of resin-linking compound-metal complex compositions*

Example 16

Preparation of sulphonated styrene-divinylbenzene resin/$[(CH_3)_2N]_3P$/rhodium complex composition

The rhodium dimer $[Rh(CO)_2Cl]_2$ (0.66 g, 1.7 mmol.) was dissolved in 85 ml of benzene in 100 ml round-bottomed flask, 1.5 g of the resin material prepared as described in Example 1 was added. The mixture was side-stirred magnetically for 1.5 hours filtered by suction, and the resin washed with 3 x 50 ml of benzene. The resultant composition was dried under vacuum in an oven (45°C) to give a material analyzed by neutron activation to have 8.4%w Rh and 2.9%w P. Analysis showed a composition having the approximate formula

$$(styrene\text{-}divinylbenzene)\text{-}(SO_3^-)_{1.5}[([(CH_3)_2N]_3P)(H^+)_{1.5}][Rh(CO)_2Cl]_{0.9}.$$

Example 17

Preparation of sulphonated styrene-divinylbenzene resin/$[(CH_3)_2NC_6H_4]_3P$/rhodium complex composition

A 2.0 g sample of the aminophosphine resin material prepared in Example 2 was added to about 25 ml of dry benzene in a 500-ml flask. To this mixture was added 0.12 g (0.31 mmol.) of $(Rh(CO)_2Cl)_2$. The entire reaction sequence was performed in a helium-filled dry box.

The mixture was stirred for a 1.5 hour period and filtered to collect the composition which was washed with dry benzene and allowed to dry. The composition was stirred in a tightly capped bottle in the dry box prior to use. Analysis showed a composition having the approximate formula

$$(styrene\text{-}divinylbenzene)\text{-}(SO_3^-)_{1.5}[([(CH_3)_2NC_6H_4]_3)(H^+)_{1.5}][Rh(CO)_2Cl]_{0.4}.$$

Example 18

Preparation of sulphonated styrene-divinylbenzene resin/$[(CH_3)_2NC_6H_4]_3P$/rhodium complex composition

A 0.3 g sample of the aminophosphire/resin material prepared in Example 2 was treated in a similar manner as described in Example 17 except that 0.28 g (0.3 mmol.) of $Rh(\varphi_3P)_3Cl$ was used as the metal source. Analysis showed the composition having the approximate formula

$$(styrene\text{-}divinylbenzene)\text{-}(SO_3^-)\ [([(CH_3)_2NC_6H_4]_3P)_x(H^+)_{1.5}]\ (RhCl[\varphi_3P]_{3-x})_{0.04}.$$

where $x = 1.3$

because the aminophosphine can replace 1, 2 or 3 of the triphenyl phosphine ligands on the metal complex.

Example 19

Preparation of sulphonated styrene-divinylbenzene resin/$[(CH_3)_2NCH_2CH_2O]_3P$/rhodium complex composition

This material was prepared in a similar manner as in Example 16 except that 0.61 g (1.6 mmol.) of rhodium dimer $(Rh(CO_2)Cl)_2$ and the aminophosphine/resin material prepared as described in Example 3 were used. Analysis showed the composition having the approximate formula

$$(styrene\text{-}divinylbenzene)\text{-}(SO_3^-)_{1.5}[([(CH_3)_2NCH_2CH_2O]_3P(H^+)_{1.5}][Rh(CO)_2Cl]_{0.8}.$$

Example 20

Preparation of sulphonated styrene-divinylbenzene resin/$[\varphi_2PCH_2CH_2)_2NCH_2CH_3]$/rhodium complex composition

This material was prepared in a similar manner as in Example 16 except that 0.48 g (1.23 mmol.) of $[Rh(CO)_2Cl)_2$ and the aminophosphine resin material prepared as described in Example 4 were used. Analysis showed the composition as having the approximate formula

$$(styrene\text{-}divinylbenzene)\text{-}(SO_3^-)_{1.5}[([(C_6H_5)_2PCH_2CH_2]_2NCH_2CH_3)(H^+)_{1.5}][Rh(CO)_2Cl]_{0.3}.$$

Example 21

Preparation of sulphonated styrene-divinylbenzene resin/methyl-quaternised $[(CH_3)_2N]_3P$/rhodium complex composition

This material was prepared in a similar manner as in Example 16 except that 0.68 g (1.7 mmol.) of $[Rh(CO)_2Cl]_2$ and 3.0 g of the aminophosphine resin material prepared as described in Example 5 were used. Analysis showed the composition as having the approximate formula

$$(styrene\text{-}divinylbenzene)\text{-}(SO_3^-)\ [([(CH_3)_2N]_3P)(CH_3^+)][Rh(CO)_2Cl]_{1.4}.$$

Example 22

Preparation of sulphonated styrene-divinylbenzene resin/methyl-quaternized ($[(CH_3)_2NC_6H_4]_3P$)/rhodium complex composition

The aminophosphine/resin material prepared in Example 6 (2.0 g) was treated with 0.17 g (0.43 mmol.) of $[Rh(CO)_2Cl]_2$ in benzene solution in a helium-filled dry box in a manner similar to that described in Example 17.

Analysis showed the composition as having the approximate formula

$$(styrene\text{-}divinylbenzene)\text{-}(SO_3^-)[([(CH_3)_2NC_6H_4]_3P)(CH_3^+)][Rh(CO)_2Cl]_{0.5}.$$

Example 23

Preparation of styrene-divinylbenzene resin/methyl quaternized $[(CH_3)_2NCH_2CH_2O]_3P$/rhodium complex compostion

This material was prepared in a similar manner as in Example 16 except that 0.08 g (0.21 mmol.) of $[Rh(CO)_2Cl]_2$ and the aminophosphine resin material prepared as described in Example 7 were used. Analysis showed the composition as having the approximate formula

$$(styrene\text{-}divinylbenzene)\text{-}(SO_3^-)[([(CH_3)_2NCH_2CH_2O]_3P)(CH_3^+)][Rh(CO)_2Cl]_6.$$

Example 24

Preparation of sulphonated styrene-divinylbenzene resin/methyl-quaternized ($[(C_6H_5)_2PCH_2CH_2]_2NCH_2CH_3$)/rhodium complex compound

This material was prepared in a similar manner as in Example 16 except that 0.45 g (1.2 mmol.) of the $[Rh(CO)_2Cl]_2$ rhodium and 1.2 g of the aminophosphine/resin material prepared as described in Example 8 were used. Analysis showed a composition having the approximate formula

$$(styrene\text{-}divinylbenzene)\text{-}(SO_3^-)[([(C_6H_5)_2PCH_2CH_2]_2NCH_2CH_3)(CH_3^+)][Rh(CO)_2Cl]_{0.5}.$$

Example 25

Preparation of sulphonated styrene resin/methyl-quaternized $([(CH_3)_2NC_6H_4]_3P$/rhodium complex composition

This material was prepared in a manner similar to Example 19 except that 0.26 g (0.68 mmol.) of $[Rh(CO)_2Cl]_2$ and 1.2 g of the aminophosphine/resin material prepared as described in Example 10 were used. Analysis showed the composition as having the approximate formula

$$(styrene)\text{-}(SO_3^-)[([(CH_3)_2NC_6H_4]_3P)(CH_3^+)][Rh(CO)_2Cl]_{0.01}.$$

Example 26

Preparation of phosphonated styrene-divinylbenzene resin/methyl-quaternized $([(CH_3)_2NC_6H_4]_3P$/rhodium complex composition

In the manner described in Example 17, a 4.5 g sample of the aminophosphine/resin material prepared in Example 17, a 4.5 g sample of the aminophosphine/resin material prepared in Example 11 was treated with 0.26 g (0.68 mmol.) of $[Rh(CO)_2Cl]_2$. Analysis showed a composition having the approximate formula

$$(styrene\text{-}divinylbenzene)\text{-}(PO_3^-)[([(CH_3)_2NC_6H_4]_3P)\ (CH_3^+)]\ [Rh(CO)_2Cl]_{0.1}$$

Example 27

Preparation of carboxylated acrylic resin/methyl-quaternized ($[(CH_3)_2NC_6H_4]_3P$)/rhodium complex composition

In the manner described in Example 17, a 4.5 g sample of the aminophosphine/resin material prepared in Example 12 was treated with 0.26 g (0.68 mmol.) of $[Rh(CO)_2Cl]_2$. Analysis showed the composition as having the approximate formula

$$(acrylic)\text{-}(CO_2^-)[([(CH_3)_2NC_6H_4]_3P)(CH_3^+)][Rh(CO)_2Cl]_{0.01}.$$

Example 28

Preparation of vinylpyridinium resin/$[o^-\text{-}O_2CC_6H_5P(C_6H_5)_2]$/rhodium complex composition

In the manner described in Example 17, a 2.4 g sample of the phosphinobenzoic acid/resin material prepared in Example 13 was treated with 0.14 g (0.34 mmol.) of $[Rh(CO)_2Cl]_2$. Analysis showed the composition having the approximate formula

$$(ethylene)\text{-}(pyridinium^+)[o^-\text{-}O_2CC_6H_5P(C_6H_5)_2][Rh(CO)_2Cl]_{0.05.}$$

Example 29

Preparation of sulphonated styrene-divinylbenzene resin/methyl-quaternized ($[(CH_3)_2NC_6H_4]_3P$)/rhodium complex composition

In the manner described in Example 17, a 0.5 g sample of the aminophosphine/resin material prepared in Example 6 was treated with 0.44 g (0.48 mmol.) $Rh(\varphi_3P)_3Cl$. Analysis showed the composition as having the approximate formula

$$(styrene\text{-}divinylbenzene)\text{-}(SO_3^-)_x[([(CH_3)_2NC_6H_4]_3P)(CH_3^+)]_x(RhCl\ [P(C_6H_5)_3]_{3-x})_{0.05},$$

where x = 1, 2 or 3.

Example 30

Preparation of sulphonated styrene-divinylbenzene resin/$[(CH_3)_2N]_3P$/cobalt complex composition

This material was prepared in a similar manner as in Example 16 except that the cobalt dimer $Co_2(CO)_8$ (0.58 g, 1.7 mmol.) was used instead of the rhodium dimer. Analysis showed the composition as having the approximate formula

(styrene-divinylbenzene)-$(SO_3^-)_{1.5}[([(CH_3)N]_3P)(H^+)_{1.5}][Co(CO)_4]_{0.5}$.

Example 31

Preparation of sulphonated styrene-divinylbenzene resin/$[(CH_3)_2NC_6H_4]_3P$/cobalt complex composition

This material was prepared in a similar manner as in Example 17 except that the complex dicobalt octacarbonyl (0.5 g, 1.45 mmol.) and 2.0 g of the aminophosphine/resin material prepared as described in Example 2 were used. Analysis showed the composition as having the approximate formula

(styrene-divinylbenzene)-$(SO_3^-)_{1.5}[([(CH_3)_2NC_6H_4]_3P)(H^+)_{1.5}][Co(CO)_4]_{0.8}$.

Example 32

Preparation of sulphonated styrene-divinylbenzene resin/$[(CH_3)_2NCH_2CH_2O]_3P$/cobalt complex composition

This material was prepared in a similar manner as in Example 19 except that the complex $Co_2(CO)_8$ (0.54 g, 1.6 mmol.) was used. Analysis showed the composition as having the approximate formula

(styrene-divinylbenzene)-$(SO_3^-)_{1.5}$ $[([(CH_3)_2NCH_2CH_2O]_3P)(H^+)_{1.5}][Co(CO)_4]_{0.3}$

Example 33

Preparation of sulphonated styrene-divinylbenzene resin/$[(\varphi_2PCH_2CH_2)_2NCH_2CH_3]$/cobalt complex composition

The material was prepared in a similar manner as in Example 20 except that the complex $Co_2(CO)_8$ (0.42 g. 1.3 mmol.) was used. Analysis showed the compositions as having the approximate formula

(styrene-divinylbenzene)-$(SO_3^-)_{1.5}[([(C_6H_5)_2PCH_2CH_2]_2NCH_2CH_3)(H^+)_{1.5}][Co(CO)_4]_{0.2}$.

Example 34

Preparation of sulphonated styrene-divinylbenzene resin/methyl-quaternized $[(CH_3)_2N]_3P$/cobalt complex composition

This material was prepared in a similar manner as in Example 21 except that the complex $Co_2(CO)_8$ (0.6 g, 1.7 mmol.) and 24 g of the aminophosphine/resin material prepared as described in Example 5 were used. Analysis showed the composition as having the approximate formula

(styrene-divinylbenzene)-$(SO_3^-)[([(CH_3)_2N]_3P)(CH_3^+)][Co(CO)_4]_{0.8}$.

Example 35

Preparation of sulphonated styrene-divinylbenzene resin/methyl-quaternized $([(CH_3)_2NC_6H_4]_3P)$/cobalt complex composition

This material was prepared in a similar manner as in Example 22 except that the complex $Co_2(CO)_8$ (0.14 g, 0.4 mmol). and 20 g of the aminophosphine resin material prepared as described in Example 6 were used. Analysis showed the composition as having the approximate formula

(styrene-divinylbenzene)-$(SO_3^-)[([(CH_3)_2NC_6H_4]_3P)(CH_3^+)][Co(CO)_4]_{1.8}$.

Example 36

Preparation of sulphonated styrene-divinylbenzene resin/methyl-quaternized $[(CH_3)_2NCH_2CH_2O]_3P$/cobalt complex composition

This material was prepared in a similar manner as in Example 23 except that the complex $Co_2(CO)_8$ (0.07 g, 0.2 mmol.) was used. Analysis showed the composition as having the approximate formula

(styrene-divinylbenzene)-$(SO_3^-)[([(CH_3)_2NCH_2CH_2O]_3P)(CH_3^+)][Co(CO)_4]_2$.

Example 37

Preparation of sulphonated styrene-divinylbenzene resin/methyl-quaternized $([(C_6H_5)_2PCH_2CH_2]_2NCH_2CH_3)$/cobalt complex composition

This material was prepared in a similar manner as in Example 24 except that the complex $Co_2(CO)_8$ (0.4 g, 1.2 mmol.) was used. Analysis showed the composition of having the approximate formula

(styrene-divinylbenzene)-$(SO_3^-)[([(C_6H_5)_2PCH_2CH_2]_2NCH_2CH_3)(CH_3^+)][Co(CO)_4]_{0.3}$.

Example 38

Preparation of sulphonated styrene-divinylbenzene resin/$[(CH_3)_2NC_6H_4]_3P$/ruthenium complex composition

This material was prepared in a similar manner as in Example 17. except that the ruthenium complex $Ru(\varphi_3P)_3Cl_2$ (1.0 g, 1.1 mmol.) and 1.0 g of aminophosphine/resin material prepared as described in Example 2 were used. Analysis showed the composition as having the approximate formula

$$(\text{styrene-divinylbenzene})\text{-}(SO_3^-)_{1.5x}[([(CH_3)_2NC_6H_4]_3P)_x(H^+)_{1.5x}]\ (RuCl_2[P(C_6H_5)_3]_{3-x})_{0.2}$$

where x = 1, 2 or 3.

Example 39

Preparation of sulphonated styrene-divinylbenzene resin/methyl-quaternized ($[(CH_3)_2NC_6H_4]_3P$)/ruthenium complex composition

This material was prepared in a similar manner as in Example 22 except that the ruthenium complex $Ru(\varphi_3P_3)_3Cl_2$ (0.19 g, 0.2 mmol.) and 1.0 g of aminophosphine/resin material prepared as described in Example 6 were used. Analysis showed the composition as having the approximate formula

$$(\text{styrene-divinylbenzene})\text{-}(SO_3^-)_x[([(CH_3)_2NC_6H_4]_3P)(CH_3^+)]_x\ (RuCl_2\ [P(C_6H_5)_3]_{3-x})_{0.05}$$

where x = 1, 2 or 3.

Example 40

Preparation of sulphonated styrene-divinylbenzene resin/$[(CH_3)_2N]_3P$/ruthenium complex composition

This material was prepared in a similar manner as in Example 16 except that the ruthenium complex $Ru(CO)_3(\varphi_3P)_2$ (1.1 g, 1.6 mmol.) was used. Analysis showed the composition as having the approximate formula

$$(\text{styrene-divinylbenzene})\text{-}(SO_3^-)_{1.5x}[([(CH_3)_2N]_3P)_x(H^+)_{1.5x}](Ru(CO)_3[P(C_6H_5)_3]_{2-x})_{0.05}$$

where x = 1 or 2.

Example 41

Preparation of sulphonated styrene-divinylbenzene methyl-quaternized ($[(CH_3)_2NC_6H_4]_3P$)/ruthenium complex composition

This material was prepared in a similar manner as in Example 39 except that the ruthenium complex $Ru(CO)_3(\varphi_3P)_2$ (0.77 g, 1.1 mmol.) was used. Analysis showed the composition as having the approximate formula

$$(\text{styrene-divinylbenzene})\text{-}(SO_3^-)_x[([(CH_3)_2NC_6H_4]_3P)(CH_3^+)]_x\ (Ru(CO)_3[P(C_6H_5)_3]_{2-x})_{0.04}$$

where x = 1 or 2.

Example 42

Preparation of sulphonated styrene-divinylbenzene resin/$[(CH_3)_2NCH_2CH_2O]_3P$/ruthenium complex composition

This material was prepared in a similar manner as in Example 19 except that the ruthenium complex $Ru(CO_3)(\varphi_3P)_2$ (0.15 g, 0.21 mmol.) was used. Analysis showed the composition as having the approximate formula

$$(\text{styrene-divinylbenzene})\text{-}(SO_3^-)_{1.5x}[([(CH_3)_2NCH_2CH_2O]_3P)_x(H^+)_{1.5x}](Ru(CO)_3[P(C_6H_5)_3]_{2-x})_{0.04}$$

where x = 1 or 2.

Example 43

Preparation of sulphonated styrene-divinyl benzene resin/$[(\varphi_2PCH_2CH_2)_2NCH_2CH_3]$/ruthenium complex composition

This material was prepared in a similar manner as in Example 20 except that the ruthenium complex $Ru(CO)_3(\varphi_3P)_2$ (0.87 g, 1.2 mmol.) was used. Analysis showed the composition as having the approximate formula

$$(\text{styrene-divinylbenzene})\text{-}(SO_3^-)_x[C_6H_5)_2PCH_2CH_2]_2NCH_2CH_3)(H^+)]_x\ (Ru(CO)_3\ [P(C_6H_5)_3]_{2-x})_{0.04}$$

where x = 1 or 2.

Example 44

Preparation of sulphonated styrene-divinylbenzene resin/methyl-quaternized $[(CH_3)_2N]_3P$/ruthenium complex composition

This material was prepared in a similar manner as in Example 21 except that the ruthenium complex $Ru(CO)_3(\varphi_3P)_2$ (1.23 g, 1.7 mmol.) was used. Analysis showed the composition as having the approximate formula

$$(\text{styrene-divinylbenzene})\text{-}(SO_3^-)_x[([(CH_3)_2N]_3P)(CH_3^+)]_x\ (Ru(CO)_3[P(C_6H_5)_3]_{2-x})_{0.04}$$

where x = 1 or 2.

Example 45

Preparation of sulphonated styrene-divinylbenzene resin/methyl-quaternized $[(CH_3)_2NC_6H_4]_3P$/ruthenium complex composition

This material was prepared in a similar manner as in Example 22 except that the ruthenium complex $Ru(CO)_3(P\varphi_3)_2$ (0.14 g, 0.2 mmol.) and 1.0 g of the aminophosphine resin material prepared as described in Example 6 were used. Analysis showed the composition as having the approximate formula

(styrene-divinylbenzene)-$(SO_3^-)_x[([(CH_3)_2NC_6H_4]_3P)(CH_3^+)]_x$ $(Ru(CO)_3$ $[P(C_6H_5)_3]_{3-x})_{0.01}$

where x = 1 or 2.

Example 46

Preparation of sulphonated styrene-divinylbenzene resin/methyl-quaternized $[(CH_3)_2NCH_2CH_2O]_3P$/ruthenium complex composition

This material was prepared in a similar manner as in Example 23 except that the ruthenium complex $Ru(CO)_3(\varphi_3P)_2$ (0.15 g, 0.2 mmol.) was used. Analysis showed the composition as having the approximate formula

(styrene-divinylbenzene)-$(SO_3^-)$ $[([(CH_3)_2NCH_2CH_2O]_3P)(CH_3^+)]_x(Ru(CO)_3[P(C_6H_5)_3]_{2-x})_{0.01}$

where x = 1 or 2.

Example 47

Preparation of sulphonated styrene-divinylbenzene resin/methyl-quaternized $([(C_6H_5)_2PCH_2CH_2]_2NCH_2CH_3)$/ruthenium complex composition

This material was prepared in a similar manner as in Example 24 except that the ruthenium complex $Ru(CO)_3(\varphi_3P)_2$ (0.82 g, 1.2 mmol.) was used. Analysis showed the composition as having the approximate formula

(styrene-divinylbenzene)- $(SO_3^-)_x[(([(C_6H_5)_2PCH_2CH_2]_2NCH_2CH_3)(CH_3^+)]_x(Ru(CO)_3[P(C_6H_5)_3]_{2-x})_{0.01}$

where x = 1 or 2.

Example 48

Preparation of sulphonated styrene-divinylbenzene resin/methyl-quaternized $([(CH_3)_2NC_6H_4]_3P$/molybdenum complex composition

To a 1-litre flask was added 1.73 g $Mo(NO)_2Cl_2$, 50 ml of methanol, and 15 g of aminophosphine/resin material prepared as described in Example 6. The mixture was stirred for 24 hours, the solid filtered and washed with methanol and dried under vacuum. Analysis showed the composition as having the approximate formula

(styrene-divinylbenzene)-$(SO_3^-)_x$ $[([(CH_3)_2NC_6H_4]_3P)(CH_3^+)]_x[Mo(NO)_{2-x}Cl_{0.4}]_{0.3}$

where x = 1 or 2.

Example 49

Preparation of sulphonated styrene-divinylbenzene resin/$[(CH_3)_2NC_6H_4]_3P$/palladium complex composition

The aminophosphine/resin material (0.3 g) prepared in Example 2 was treated with 0.12 g (0.3 mmol.) of Pd $(\varphi CN)_2CL_2$ in a manner similar to that described in Example 17. Analysis showed the composition having the approximate formula

(styrene-divinylbenzene)-$(SO_3^-)_{1.5x}[([(CH_3)_2NC_6H_4]_3P)_x$ $(H^+)_{1.5x}]$ $[Pd(C_6H_5CN)_{2-x}Cl_2]_{0.5}$

where x = 1 or 2.

Example 50

Preparation of sulphonated styrene-divinylbenzene resin/methyl-quaternized $[(CH_3)_2NC_6H_4]_3P$/palladium complex composition

The aminophosphine/resin material (0.5 g) prepared in Example 6 was treated with 0.18 g (0.48 mmol.) of $Pd(\varphi CN)_2Cl_2$ in a manner similar to that described in Example 17. Analysis showed the composition as having the approximate formula

(styrene-divinylbenzene)-$(SO_3^-)_x[([(CH_3)_2NC_6H_4]_3P)(CH_3^+)]_x$ $[Pd(C_6H_5CN)_{2-x}Cl_2)]_{0.5}$

where x = 1 or 2.

Example 51

Preparation of sulphonated styrene-divinylbenzene resin/$[(CH_3)_2NC_6H_4]_3P$/palladium complex composition

To a solution of 0.73 g (1.0 mmol.) of $PdCl_2(\varphi_3P)_2$ in 70.0 ml $CHCl_3$ were added 2.0 g of the aminophosphine/resin material prepared as described in Example 2. The mixture was stirred for 1 hour, filtered, washed with $CHCl_3$, and dried under vacuum. The resultant composition was Soxhlet extracted with methyl ethyl ketone for 4 hours, and dried in vacuum oven at approximately 40°C. Analysis showed the composition as having the approximate formula

$(styrene\text{-}divinylbenzene)\text{-}(SO_3^-)_{1.5x}[([(CH_3)_2NC_6H_4]_3P)_x(H^+)_{1.5x}](PdCl_2[P(C_6H_5)_3]_{2-x})_{0.2}$
where $x = 1$ or 2.

Example 52

Preparation of sulphonated styrene-divinylbenzene resin/$[(CH_3)_2NC_6H_4]_3P$/palladium-tin complex composition

2.0 grams of aminophosphine/resin material prepared as described in Example 51 (prior to the Soxhlet-extraction step) were added to a filtered solution of 1.2 g (5.2 mmol.) of $SnCl_2.2H_2O$ in 200 ml of acetone. The mixture was stirred on side for 1 hour, filtered, washed with acetone, vacuum dried, Soxhlet-extracted with methyl ethyl ketone for 4 hours, and dried in vacuum at 40°C. Analysis showed the composition as having the approximate formula

$(styrene\text{-}divinylbenzene)\text{-}(SO_3^-)_{1.5x}[([(CH_3)_2NC_6H_4]_3P)_x(H^+)_{1.5x}]\ (SnCl_2)_{0.2x}\ (PdCl_2[P(C_6H_5)_3]_{2-x})_{0.2}$
where $x = 1$ or 2.

Example 53

Preparation of sulphonated styrene divinylbenzene resin/$[(CH_3)_2NC_6H_4]_3P$/palladium complex composition

The palladium dimer $[Pd(allyl)Cl]_2$ (1.36 g, 3.5 mmol.) was dissolved in 50 ml of toluene and stirred overnight with 5.0 g of aminophosphine/resin material prepared as described in Example 2. The resulting composition was then filtered, washed with toluene, Soxhlet-extracted with toluene for 4 hours and dried under vacuum at 50°C. Analysis showed the composition as having the approximate formula

$(styrene\text{-}divinylbenzene)\text{-}(SO_3^-)_{1.5x}[([(CH_3)_2NC_6H_4]P_3)_x(H^+)_{1.5x}]\ [PdCl(C_3H_5)_{2-x}]_{0.2}$
where $x = 1$ or 2.

Example 54

Preparation of quaternary ammonium styrene-divinylbenzene resin/$(HO_2CC_5H_4N)$/palladium complex composition

To a toluene solution of palladium acetate (1.0 g, 4.4 mmol.) were added 6 g of iso-nicotinic acid/resin material prepared as described in Example 14; the mixture stirred on the side overnight, filtered, and the resulting composition Soxhlet-extracted with toluene for 5 hours. The material was then dried in vacuum oven overnight (45°C). Analysis showed the composition as having the approximate formula

$(styrene\text{-}divinylbenzene)\text{-}[CH_2N^+(CH_3)_3]\ (^-O_2CC_5H_4N)[Pd(O_2CCH_3)_2]_{0.1}$.

Example 55

Preparation of quaternary ammonium styrene-divinylbenzene resin/$(HO_2CC_5H_4N)$/palladium complex composition

This material was prepared in a similar manner as in Example 54 except the aminophosphine/resin material was that prepared as described in Example 15. Analysis showed the composition as having the approximate formula

$(styrene\text{-}divinylbenzene)\text{-}[CH_2N^+(CH_3)_3](^-O_2CC_5H_4N)\ [Pd(O_2CCH_3)_2]_{0.1}$.

Example 56

Preparation of sulphonated styrene-divinylbenzene resin/$[(CH_3)_2NC_6H_4]_3P$/platinum-tin-complex composition

To a 90 ml acetone solution of the platinum complex $PtH(SnCl_3)(\varphi_3P)_2$ (1.2 g, 1.2 mmol.) were added 2.0 g of the aminophosphine/resin material prepared as described in Example 2, side-stirred magnetically for 1.5 hours, filtered, the resultant composition washed with acetone, and vacuum dried at 45°C. Analysis showed the composition as having the approximate formula

$(styrene\text{-}divinylbenzene)\text{-}(SO_3^-)_{1.5x}[([(CH_3)_2NC_6H_4]_3P)(H^+)]_{1.5x}(PtH(SnCl_3)\ [P(C_6H_5)_3]_{2-x})_{0.1}$
where $x = 1$ or 2.

Example 57

Preparation of sulphonated styrene-divinylbenzene resin/methyl-quaternized $([(CH_3)_2NC_6H_4]_3P)$/platinum-tin-complex composition

This material was prepared as in Example 56 except that 0.17 g (0.18 mmol.) of the platinum complex and 1.0 g of the aminophosphine/resin material prepared as described in Example 6 were used. Analysis showed the composition as having the approximate formula

$(styrene\text{-}divinylbenzene)\text{-}(SO_3^-)_x\ [([(CH_3)_2NC_6H_4]_3P)(CH_3^+)]_x(PtH(SnCl_3)\ [P(C_6H_5)_3]_{2-x})_{0.04}$
where $x = 1$ or 2.

Example 58

Preparation of sulphonated styrene-divinylbenzene resin/methyl-quaternized ([$CH_3$)$_2NC_6H_4$]$_3$P)/platinum-tin-complex composition

This material was prepared in a similar manner as in Example 56 except that the platinum complex PtH(CO)($SnCl_3$) ($\varphi_2$P)$_2$ was used instead and 200 ml of acetone were used. Analysis showed the composition as having the approximate formula

$$\text{(styrene-divinylbenzene)-}(SO_3^+)_{1.5x}[([(CH_3)_2NC_6H_4]_3P)\ (CH_3^+)]_{1.5x}(PtH(SnCl_3)\ [P(C_6H_5)_3]_{2-x})_{0.04}$$

where x = 1 or 2.

Example 59

Preparation of sulphonated styrene-di-vinylbenzene resin/ [($CH_3$)$_2NC_6H_4$]$_3$P/platinum-tin-complex composition

To a 50 ml benzene solution of [$PtCl_2(Bu_3P)$]$_2$ (1.2 g, 1.2 mmol.) were added 2.0 g of aminophosphine/resin material prepared as described in Example 2, the mixture stirred on the side magnetically for 1.5 hours, and filtered. The resultant filtrate was passed through the resin material on the filter by gravity three times, filtered, and then washed with benzene, and vacuum-dried. The resin material was then added to a 400 ml acetone solution of $SnCl_2.2H_2O$ (2.5 g), side-stirred for 40 minutes, filtered, washed with acetone, and vacuum-dried. Analysis showed the composition as having the formula

$$\text{(styrene-divinylbenzene)-}(SO_3^-)_{1.5}[([(CH_3)_2NC_6H_4]_3P)(H^+)_{1.5}](SnCl_2)0.2[PtCl_2\ (n\text{-}C_4H_{10})_3P])_{0.2}.$$

Example 60

Preparation of sulphonated styrene-divinylbenzene resin/methyl-quaternized ([($CH_3$)$_2NC_6H_4$]$_3$P)/platinum-tin-complex composition

This material was prepared as described in Example 59 except that 0.33 g (0.36 mmol.) of [$PtCl_2(Bu_3P)$]$_2$ was used and the aminophosphine/resin material was then prepared as described in Example 6. Analysis showed the composition as having the formula

$$\text{(styrene-divinylbenzene)-}(SO_3^-)[([(CH_3)_2NC_6H_4]_3P)\ (CH_3^+)]\ (SnCl_2)_{0.2}PtCl_2[(n\text{-}C_4H_{10})_3P])_{0.05}.$$

*Processes utilizing compositions of the invention*

Example 61

Hydroformylation process

To a 300 ml autoclave were added 70 ml of benzene, 2.0 ml of n-decane (internal standard), 20.0 ml (160 mmol.) of 1-hexane, and 0.5 g of the resin/ligand/cobalt catalyst as described in Example 31. The solution was deoxygenated with nitrogen. Synthesis gas ($CO/H_2$, 1:1, (84 bar) 1200 psig) was then charged to the reactor and the reactor is heated to 120°C. After 20 hours, gas chromatographic analysis revealed the following results:

| | |
|---|---|
| Conversion (mol.%) | 47 |
| Selectivity to $C_7$-aldehyde (mol.%) | 99 |
| Selectivity to hexane (mol.%) | 1 |
| Linearity of $C_7$-aldehyde | 71 |
| Reaction rate (mol./mol./h) | 24 |

Example 62

Hydrogenation process

To a 300 ml autoclave were added 80 ml of toluene, 2.0 ml of n-decane (internal standard), 10.0 ml (80 mmol.) of 1-hexene, and 1.7 g of the resin/ligand/rhodium catalyst as described in Example 17. The solution was deoxygenated with nitrogen, and 34 bar (500 psig) of hydrogen were charged to the reactor. The reactor was then heated to 100°C. After 1 hour, gas chromatographic analysis revealed the following results:

| | |
|---|---|
| Conversion (mol.%) | 67 |
| Selectivity to n-hexane (mol.%) | 97 |
| Reaction rate (mol./mol./h) | 170 |

Example 63

Olefin metathesis (disproportionation) process

To a mixture of 4 ml of chlorobenzene, 2 ml of 1-hexane, 1.0 g of the resin/ligand-molybdenum composition prepared as described in Example 48 were added to 200 ml of 25% $Al_2(CH_3)_3Cl_3$ in hexane. The mixture was stirred for 2 hours at room temperature. Analysis showed a 4.7% yield of decenes after 2 hours.

**Claims**

1. A complex composition characterized by:
(a) an ion exchange resin;
(b) a transition metal, and
(c) an organic linking compound which has at least one moiety which is ionically bound to said ion exchange resin and further has at least one moiety which is coordinately bound to said metal.

2. A composition as claimed in claim 1, characterized in that the ion exchange resin is a strongly acidic, weakly acidic or mixed acid-base type and the linking compound contains from 1 to about 100 carbon atoms, the ionically bound moiety of the linking compound is selected from the group consisting of monohydrocarbyl ammonium, dihydrocarbyl ammonium, trihydrocarbyl ammonium, quaternary ammonium, pyridinium, phosphonium, arsonium and sulphonium ion and the coordinately bound moiety contains a hetero-atom selected from the group consisting of trivalent nitrogen, trivalent phosphorus, trivalent arsenic, trivalent bismuth and trivalent antimony.

3. A composition as claimed in claim 2, characterized in that the functional group of the ion exchange resin is derived from sulphonic acid, fluorinated alkyl sulphonic acid, phosphonic acid, carboxylic acid or iminocarboxylic acid.

4. A composition as claimed in claim 3, characterized in that the ion exchange resin has a backbone of polystyrene, polystyrene cross-linked with divinyl benzene, phenol-formaldehyde condensate, benzene-formaldehyde condensate, polyacrylic acid or polymethacrylic acid.

5. A composition as claimed in claim 1, characterized in that the ion exchange resin is a basic-type resin, the linking compound contains from 1 to about 100 carbon atoms, the ionically bound moiety of the linking compound is derived from the group consisting of carboxylic acid, phosphonic acid, phosphinic acid, sulphenic acid, sulphinic acid, sulphonic acid, boronic acid and boronous acid and the coordinately bound moiety contains a hetero-atom selected from the group consisting of trivalent nitrogen, trivalent phosphorus, trivalent arsenic, trivalent bismuth and trivalent antimony.

6. A composition as claimed in claim 5, characterized in that the functional group of the ion exchange resin is selected from the group consisting of primary, secondary, tertiary, quaternary amine and pyridinium.

7. A composition as claimed in claim 6, characterized in that the ion exchange resin has a backbone of polystyrene, polystyrene cross-linked with divinyl benzene, phenol-formaldehyde condensate, benzene-formaldehyde condensate, epoxypolyamine and phenolic polyamine.

8. A composition as claimed in claims 1—7, characterized in that the transition metal is from Group VIB, VIIB, VIII or IB.

9. A process for hydroformylating olefins with hydrogen and carbon monoxide, characterized in that is used as a catalyst a composition as claimed in claims 1—8, wherein the transition metal is cobalt, ruthenium, palladium, platinum or rhodium.

10. A process for carbonylating olefins with carbon monoxide and a hydroxylic compound, characterized in that is used as a catalyst a composition as claimed in claims 1—8, wherein the transition metal is cobalt, ruthenium, palladium, platinum or rhodium.

11. A process for hydrogenating an organic compound, characterized in that is used as a catalyst a composition as claimed in claims 1—8, wherein the transition metal is cobalt, ruthenium, platinum, palladium or rhodium.

12. A process of isomerizing organic compounds, characterized in that is used as a catalyst a composition as claimed in claims 1—8, wherein the transition metal is cobalt, molybdenum, ruthenium, palladium, platinum or rhodium.

13. A process of disproportionating olefins, characterized in that is used as a catalyst a composition as claimed in claims 1—8, wherein the transition metal is molybdenum, tungsten or rhenium.

14. A process of oligomerizing organic compounds, characterized in that is used as a catalyst a composition as claimed in claims 1—8, wherein the metal is nickel, palladium or platinum.

15. A process of preparing hydrocarbons by reacting hydrogen and carbon monoxide, characterized in that is used as a catalyst a composition as claimed in claims 1—8, wherein the metal is ruthenium or rhodium.

**Revendications**

1. Composition complexe caractérisée par
(a) une résine échangeuse d'ion;
(b) un métal de transition; et
(c) un composé de liaison organique dont au moins une fraction est liée de façon ionique à ladite résine échangeuse d'ions et dont en outre au moins une fraction est liée par coordination audit métal.

2. Composition telle que revenidiquée dans la revendication 1, caractérisée en ce que la résine échangeuse d'ions est du type fortement acide, faiblement acide ou acid-base mixte et en ce que le composé de liaison contient de 1 à environ 100 atomes de carbone, la fraction liée de façon ionique du composé de liaison étant choisie dans le groupe constitué par les ions monohydrocarbylammonium, dihydrocarbylammonium, trihydrocarbylammonium, ammonium quaternaire, pyridinium, phosphonium, arsonium et sulfonium et la fraction liée par coordination contenant un hétéroatome choisi dans le groupe constitué par l'azote trivalent, le phosphore trivalent, l'arsenic trivalent, le bismuth trivalent et l'antimoine trivalent.

3. Composition tell que revendiquée dans la revendication 2, caractérisée en ce que le groupe fonctionnel de la résine échangeuse d'ions est dérivé d'un acid sulfonique, d'un acide alcoylsulfonique d'un acide phosphonique, d'un acide carboxylique ou d'un acide iminocarboxylique.

4. Composition tell que revendiquée dans la revendication 3, caractérisée en ce que la résine échangeuse d'ions a une ossature de polystyrène, de polystyrène réticulé avec du divinylbenzène, de condensat phénol-formaldéhyde, de condensat benzène-formaldéhyde, d'acide polyacrylique ou d'acide polyméthacrylique.

5. Composition telle que revendiquée dans la revendication 1, caractérisée en ce que la résine échangeuse d'ions est une résine du type basique, le composé de liaison contient d'environ 1 à environ 100 atomes de carbone, la fraction liée de façon ionique du composé de liaison est dérivée du groupe constitué par les acides carboxyliques, phosphonique, phosphinique, sulfénique, sulfinique, sulfonique, boronique et boroneux et la fraction liée par coordination contient un hétéro-atome choisi dans le groupe constitué par l'azote trivalent, le phosphore trivalent, l'arsenic trivalent, le bismuth trivalent et l'antimoine trivalent.

6. Composition telle que revendiquée dans la revendication 5, caractérisée en ce que le groupe fonctionnel de la résine échangeuse d'ions est choisie dans le groupe constitué par les amines primaire, secondaire, tertiaire et quatemaire et le pyridinium.

7. Composition telle que revendiquée dans la revendication 6, caractérisée en ce que la résine échangeuse d'ions a une ossature de polystyrène, de polystyrène réticulé avec du divinylbenzène, de condensat de phénol-formaldéhyde, de condensat de benzène-formaldéhyde, d'époxypolyamine et de polyamine phénolique.

8. Composition telle que revendiquée dans les revendications 1 à 7, caractérisée en ce que le métal de transition est du groupe VIB, VIIB, VIII ou IB.

9. Procédé d'hydroformylation des oléfines avec de l'hydrogène et du monoxyde de carbone, caractérisé en ce que qu'on utilise comme catalyseur une composition telle que revendiquée dans les revendications 1 à 8, où le métal de transition est le cobalt, le ruthénium, le palladium, le platine ou le rhodium.

10. Procédé de carbonylation des oléfines avec du monoxyde de carbone et un composé hydroxylique, caractérisé en ce qu'on utilise comme catalyseur une composition telle que revendiquée dans les revendications 1 á 8, où le métal de transition est le cobalt, le ruthénium, le palladium, le platine ou le rhodium.

11. Procédé d'hydrogénation d'un composé organique, caractérisé en ce qu'on utilise comme catalyseur une composition telle que revendiquée dans les revendications 1 à 8, où le métal de transition est le cobalt, le ruthénium, le platine, le palladium ou le rhodium.

12. Procédé d'isomérisation des composés organiques, caractérisé en ce qu'on utilise comme catalyseur une composition telle que revendiquée dans les revendications 1 à 8, où le métal de transition est le cobalt, le molybdène, le ruthénium, le palladium, le platine ou le rhodium.

13. Procédé de dismutation des oléfines, caractérisé en ce qu'on utilise comme catalyseur une composition telle que revendiquée dans les revendications 1 à 8, où le métal de transition est le molybdène, le tungstène ou le rhénium.

14. Procédé d'oligomérisation des composés organiques, caractérisé en ce qu'on utilise comme catalyseur une composition telle que revendiquée dans les revendications 1 à 8, où le métal est le nickel, le palladium ou le platine.

15. Procédé de préparation des hydrocarbures en faisant réagir de l'hydrogène et du monoxyde de carbone, caractérisé en ce qu'on utilise comme catalyseur une composition telle que revendiquée dans les revendications 1 à 8, où le métal est le ruthénium ou le rhodium.

**Patentansprüche**

1. Eine komplexe Zusammensetzung, gekennzeichnet durch
(a) ein Ionenaustauscherharz
(b) ein Übergangsmetall und
(c) eine organische verknüpfende Verbindung, die mindestens einen Rest aufweist, der ionisch an das Ionenaustauscherharz gebunden ist, und ausserdem mindestens einen Rest hat, der koordinativ an das Metall gebunden ist.

2. Zusammensetzung gemäss Anspruch 1, dadurch gekennzeichnet, dass das Ionenaustauscherharz ein stark saures, ein schwach saures oder ein gemischtes sauer-basisches Harz ist und die verknüpfende Verbindung von 1 bis etwa 100 Kohlenstoffatome enthält, der ionisch gebundene Rest der verknüpfenden Verbindung aus der Gruppe ausgewählt ist, die aus einem mit einem Kohlenwasserstoffrest substituierten Ammoniumrest, einem mit 2 Kohlenwasserstoffresten substituierten Ammoniumrest, einem mit 3 Kohlenwasserstoffresten substituierten Ammoniumrest, einem quartären Ammoniumrest, einem Pyridinium-, Phosphonium-, Arsonium- und Sulfonium-Ion besteht, und der koordinativ gebundene Rest ein Heteroatom enthält, welches aus der Gruppe ausgewählt ist, die aus einem trivalenten Stickstoffatom, einem trivalenten Phosphoratom, einem trivalenten Arsenatom, einem trivalenten Wismutatom und einem trivalenten Antimonatom besteht.

3. Zusammensetzung gemäss Anspruch 2, dadurch gekennzeichnet, dass die funktionelle Gruppe des Ionenaustauscherharzes sich von der Sulfonsäure, einer fluorierten Alkylsulfonsäure, der Phosphonsäure, einer Carbonsäure oder einer Iminocarbonsäure ableitet.

4. Zusammensetzung gemäss Anspruch 3, dadurch gekennzeichnet, dass das Ionenaustauscherharz ein Gerüst aus Polystyrol, mit Divinylbenzol vernetztem Polystyrol, Phenol-Formaldehyd-Kondensationsharz, Benzol-Formaldehyd-Kondensationsharz, Polyacrylsäure und Polymethacrylsäure aufweist.

5. Zusammensetzung gemäss Anspruch 1, dadurch gekennzeichnet, dass das Ionenaustauscherharz ein basisches Harz ist, die verknüpfende Verbindung von 1 bis etwa 100 Kohlenstoffatome hat, der ionisch gebundene Rest der verknüpfenden Verbindung sich von der Gruppe ableitet, die aus einer Carbonsäure, Phosphonsäure, Phosphinsäure, Sulfensäure, Sulfinsäure, Sulfonsäure, Boronsäure oder boronige Säure besteht, und der koordinativ gebundene Rest ein Heteroatom enthält, welches aus der Gruppe ausgewählt ist, die aus einem trivalenten Stickstoffatom, trivalenten Phosphoratom, trivalenten Arsenatom, trivalenten Wismutatom und trivalenten Antimonatom besteht.

6. Zusammensetzung gemäss Anspruch 5, dadurch gekennzeichnet, dass die funktionelle Gruppe des Ionenaustauscherharzes aus der Gruppe ausgewählt ist, welche aus einem primären, sekundären, tertiären und quartären Amin und Pyridinium besteht.

7. Zusammensetzung gemäss Anspruch 6, dadurch gekennzeichnet, dass das Ionenaustauscherharz ein Gerüst aus Polystyrol, mit Divinylbenzol vernetztem Polystyrol, Phenol-Formaldehyd-Kondensationsharz, Benzol-Formaldehyd-Kondensationsharz, Epoxypolyamin und phenolischem Polyamin aufweist.

8. Zusammensetzung gemäss den Ansprüchen 1 bis 7, dadurch gekennzeichnet, dass das Ubergangsmetall ein Metall der Gruppen VIB, VIIB, VIII oder IB ist.

9. Verfahren zur Hydroformylierung von Olefinen mit Wasserstoff und Kohlenmonoxid, dadurch gekennzeichnet, dass als Katalysator eine Zusammensetzung gemäss den Ansprüchen 1 bis 8 verwendet wird, wobei das Übergangsmetall Kobalt, Ruthenium, Palladium, Platin oder Rhodium ist.

10. Verfahren zur Carbonylierung von Olefinen mit Kohlenmonoxid und einer eine Hydroxylgruppe aufweisenden Verbindung, dadurch gekennzeichnet, dass als Katalysator eine Zusammensetzung gemäss den Ansprüchen 1 bis 8 verwendet wird, wobei das Übergangsmetall Kobalt, Ruthenium, Palladium, Platin oder Rhodium ist.

11. Verfahren zur Hydrierung einer organischen Verbindung, dadurch gekennzeichnet, dass als Katalysator eine Zusammensetzung gemäss den Ansprüchen 1 bis 8 verwendet wird, wobei das Übergangsmetall Kobalt, Ruthenium, Platin, Palladium oder Rhodium ist.

12. Verfahren zur Isomerisierung von organischen Verbindungen, dadurch gekennzeichnet, dass als Katalysator eine Zusammensetzung gemäss den Ansprüchen 1 bis 8 verwendet wird, wobei das Übergangsmetall Kobalt, Molybdän, Ruthenium, Palladium, Platin oder Rhodium ist.

13. Verfahren zur Disproportionierung von Olefinen, dadurch gekennzeichnet, dass als Katalysator eine Zusammensetzung gemäss den Ansprüchen 1 bis 8 verwendet wird, wobei das Übergangsmetall Molybdän Wolfram oder Rhenium ist.

14. Verfahren zur Oligomerisierung von organischen Verbindungen, dadurch gekennzeichnet, dass als Katalysator eine Zusammensetzung gemäss den Ansprüchen 1 bis 8 verwendet wird, wobei das Metall Nickel, Palladium oder Platin ist.

15. Verfahren zur Herstellung von Kohlenwasserstoffen durch Umsetzen von Wasserstoff und Kohlenmonoxid, dadurch gekennzeichnet, dass als Katalysator eine Zusammensetzung gemäss den Ansprüchen 1 bis 8 verwendet wird, wobei das Metall Ruthenium oder Rhodium ist.